# EUROPEAN PATENT APPLICATION

(11) **EP 0 680 760 A1**
(43) Date of publication of application: **08.11.1995**
(21) Application number: 95302199.5
(22) Date of filing: 31.03.1995
(51) Int. Cl.: A61K 31/665

(54) **Topical antipruritic composition containing a phosphonic acid diester compound**

(30) Priority: 05.04.1994 JP 93082/94
(71) Applicant: Senju Pharmaceutical Co., Ltd., Osaka-shi, Osaka 541 (JP)
(72) Inventor: Yoshida, Shoji, Nishinomiya, Hyogo 662 (JP); Kawahira, Osamu, Sakai, Osaka 588 (JP); Ogata, Kazumi, Toyonaka, Osaka 565 (JP)
(74) Representative: Burford, Anthony Frederick

(57) **Abstract**

Phosphoric acid diester compounds of the following formula (I) and pharmacologically acceptable salts thereof are used in a topical dosage form to contact a focus of pruritus to provide an antipruritic effect: 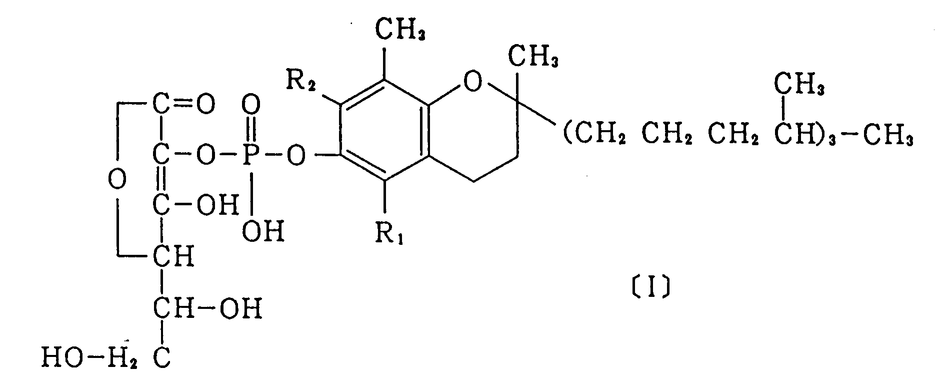
(wherein R₁ and R₂ are the same or different and each represents a hydrogen atom or a methyl group).

## Description

This invention relates to antipruritic medication. More particularly, the invention relates to an antipruritic composition comprising a phosphoric acid diester compound of the following formula (I): 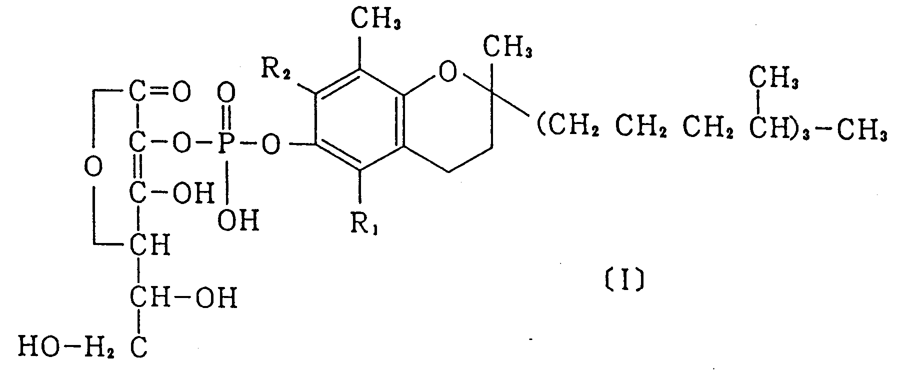
(wherein R₁ and R₂ are the same or different and each represents a hydrogen atom or a methyl group), or a pharmaceutically acceptable salt thereof.

Aqueous ammonia, menthol, camphor and the like are conventionally used for insect bites, while antihistaminics and steroids are used for prevention of the itch associated with urticaria. However, since the antipruritic effect of these remedies is only transitory, it is necessary to repeat medication and, as far as steroids are concerned, their side effects call for caution in long-term usage. Therefore, the advent of an antipruritic medicine with improved efficacy has been awaited.

Reference in this application to compounds of formula (I) includes the free compounds and pharmacologically acceptable salts thereof unless another meaning is clear from the context. The salts include alkali metal salts, such as sodium salts and potassium salts, and alkaline earth metal salts, such as calcium salts and magnesium salts.

Compounds of formula (I) are known for use as anticataract drugs, prophylactic/therapeutic drugs for climacteric disturbance, skin-care cosmetics (JP-A-2-44478), antiinflammatory drugs (JP-A-1-27044), antiulcer drugs (JP-A-63-270626), prophylactic/therapeutic drugs for ischemic organ diseases (JP-A-2-111722) and bath preparations (JP-A-5-286848).

The Inventors have found that compounds of formula (I) exhibit remarkable efficacy, when topically administered at a certain concentration in the skin itch which is frequently encountered in diabetes and liver disorder as well as in senile pruritus. This invention has been developed on the basis of the above finding.

This invention relates to an antipruritic composition comprising a phosphoric acid diester compound of the following formula (1) or a pharmacologically acceptable salt thereof as an active ingredient in a topical dosage form that allows said active ingredient to contact a focus of pruritus at a concentration of at least 0.01 (w/w) %, preferably 0.01 - 5 (w/w) %. 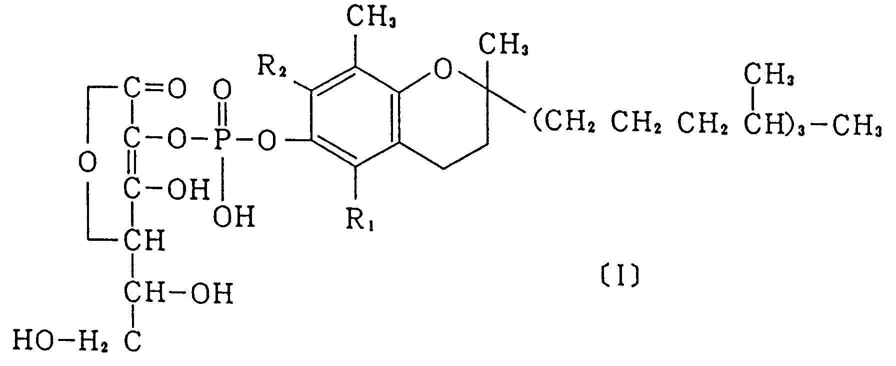
(wherein R₁ and R₂ are the same or different and each represents a hydrogen atom or a methyl group).

The compounds of formula (I) can be incorporated either singly or in combination as necessary.

The antipruritic compounds of this invention are preferably used in an ointment form or as a water-based liquid preparation. The preferred water-based liquid preparation is an aqueous spray. In the manufacture of such preparations, a variety of conventional additives such as excipients, binders, thickeners, dispersing agents, reabsorption promoters, buffers, surfactants, preservatives, isotonizing agents, stabilizers and pH control agents can be selectively employed.

The compounds of formula (I) are advantageously incorporated in an antipruritic product in such a manner that they may contact the focus of pruritus at a concentration of 0.01 - 5 (w/w) %. Below this concentration range, insufficient efficacy can be expected, while usage at any concentration over the range is not rewarded with enhanced efficacy.

The concentration of the compounds of formula (I) in the final dosage form may be 0.01 - 5 (w/w) %, preferably 0.05 - 2 (w/w) %, for an ointment or a cream, and 0.01 - 5 (w/w) %, preferably 0.05 - 2 (w/w) %, for an aqueous spray.

Unless contrary to the object of this invention, the pharmaceutical composition of this invention may be supplemented with other medicinally active substances such as antihistaminics, menthol, and camphor.

The following examples are further illustrative of this invention.
Preparation Example 1 Ointment

| | |
|---|---|
| L-Ascorbyl DL-α-tocopheryl phosphate potassium | 1.0 g |
| Hydrophilic ointment base | to 100 g |

The above materials are mixed to provide an ointment.
Preparation Example 2: Gel

| | |
|---|---|
| L-Ascorbyl DL-α-tocopheryl phosphate potassium | 0.5 g |
| 1-Menthol | 0.5 g |
| Carbopol | 1.0 g |
| Triethanolamine | q.s. |
| Ethanol | 30 ml |
| Sterilized purified water | to 100 ml |
| Adjusted to pH 7.0 | |

Preparation Example 3: Aqueous sol

| | |
|---|---|
| L-Ascorbyl DL-α-tocopheryl phospate potassium | 0.5 g |
| Glycerin | 1.0 g |
| Propylene glycol | 1.5 g |
| Ethanol | 30 ml |
| Sterilized purified water | to 100 ml |

### Example 1:

A 55-year-old man presenting with eczema accompanied by itchy sensation in the abdominal and dorsal regions due to hepatic disorder was topically treated with the drug obtained in Preparation Example 1 for 2 weeks. As a result, not only pruritus but also eczema healed almost completely.

### Example 2:

A man (58 years old) who made it a rule to have a nightcap (one pint of sake) could have a good sleep after he applied the drug of Preparation Example 3 whenever he felt an itch of the knee during the winter months. In contrast, the vehicle which did not contain the active compound was not effective.

### Example 3:

A person who had been bitten by a mosquito was topically treated with the drug of Preparation Example 1. As a result, the itch disappeared and the swelling also subsided.

## Claims

1. An antipruritic composition comprising a phosphoric acid diester compound of the following formula (I) or a pharmacologically acceptable salt thereof as an active ingredient in a topical dosage form that allows said active ingredient to contact a focus of pruritus at a concentration of at least 0/01 (w/w) %. 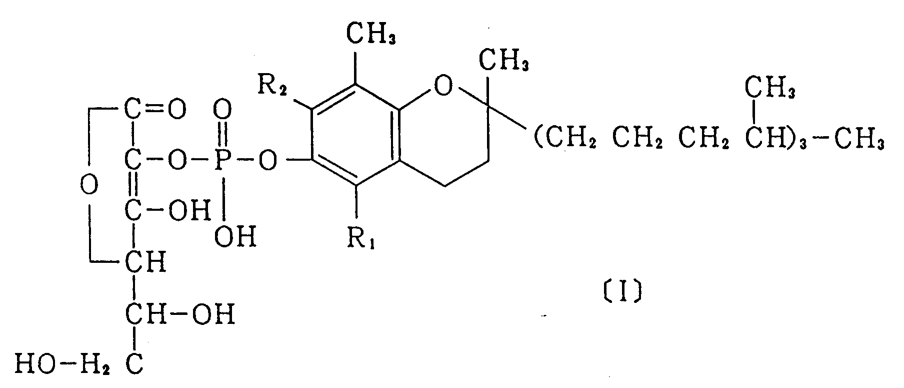 (wherein R₁ and R₂ are the same or different and each represents a hydrogen atom or a methyl group).

2. An antipruritic composition according to Claim 1, wherein said concentration is 0.01 - 5 (w/w)%.

3. An antipruritic composition according to Claim 1 or Claim 2, wherein the phosphoric acid diester is L-ascorbyl DL-α-tocopheryl phosphoric acid or a salt thereof.

4. An antipruritic composition according to any one of the preceding claims, in the form of an ointment.

5. An antipruritic composition according to any one of Claims 1 to 3, which is a water-based liquid preparation.

6. An antipruritic composition according to Claim 5, wherein said water-based liquid preparation is an aqueous spray.

7. The use of a compound of formula (I) as defined in Claim 1 or a pharmaceutically acceptable salt thereof in the manufacture of a topical medicament for the treatment of pruritus.

8. A use according to Claim 7, wherein the medicament is for the treatment of insect bites, diabetic skin itch, skin itch associated with liver disorder, skin itch associated with eczema, or senile pruritus.

9. The use of a compound of formula (I) as defined in Claim 1 or a pharmaceutically acceptable salt thereof in the manufacture of a topical medicament for the treatment of eczema.

10. A use according to any one of Claims 7 to 9, wherein the medicament contains 0.01 - 5 (w/w) % of the said compound or salt thereof.

11. A use according to any one of Claims 7 to 10, wherein the said compound is L-ascorbyl DL-α-tocopheryl phosphoric acid or a salt thereof.
